# EUROPEAN PATENT SPECIFICATION

(11) **EP 0 371 112 B1**
(45) Date of publication and mention of the grant of the patent: **14.12.1994**
(21) Application number: 89906078.4
(22) Date of filing: 26.05.1989
(51) Int. Cl.: C12N 15/76, C12N 1/20, C12P 19/56

(54) **ISOLATION AND CHARACTERISATION OF GENES RESISTANT TO ANTHRACYCLINE ANTIBIOTICS**
ISOLIERUNG UND CHARAKTERISIERUNG VON GEGEN ANTHRACYCLIN-ANTIBIOTIKA RESISTENTEN GEGEN
ISOLATION ET CARACTERISATION DE GENES RESISTANTS AUX ANTIBIOTIQUES D'ANTHRACYCLINE

(30) Priority: 27.05.1988 GB 8812697
(43) Date of publication of application: 06.06.1990
(73) Proprietor: FARMITALIA CARLO ERBA S.r.l., I-20159 Milano (IT)
(72) Inventor: CARUSO, Marinella, I-Milan (IT); COLOMBO, Anna, Luisa, I-Milan (IT); GAROFANO, Luisa, I-20052 Monza (IT); TORTI, Francesca, I-Milan (IT); ZANELLA Giuseppe, 20094 BUCCINASCO (Milan) (IT)
(74) Representative: Woods, Geoffrey Corlett
(86) International application number: EP8900588
(87) International publication number: WO8911532

(56) References cited:
- EP-A- 0 173 327
- EP-A- 0 204 549
- Experientia, vol. 38, 1982 Birkhäuser-Verlag (Basel, CH) N. Crespi-Perellino et al. pp. 1455-1456
- Abstracts of the Annual Meeting of the American Society for Microbiology, 88th Annual Meeting, 8-13 May 1988, Miami Beach, Florida, American Soc. For Microbiology (US) K.J. Stutzman-Engwall et al. s. p. 261
- 194th American Chemical Society National Meeting, 30 Aug.-4 Sept. 1987, New Orleans, Louisiana, US, Abstr. Pap. Am. Chem. Soc, 194(0) 1987, Medi 5 (US) C.R. Hutchinson et al. s. abstract
- Nature, vol. 325, 26 Febr. 1987, F. Malpartida et al. pp. 818-821
- Mol.Gen.Genet., vol. 205, 1986, Springer-Verlag T. Murakami et al. pp. 42-50
- Chemical Abstracts, vol. 104, 1986, (Columbus, Ohio, US), J.S. Lampel et al. v. p. 178, résumé 103498p & Appl. Environ. Microbiol. 1986, 51(1), 126-31
- Journal of Bacteriology, vol. 151, no. 2, (US) C.J. Thompson et al. pp. 678-685
- The Journal of Antibiotics, vol. 59, no. 1, January 1986 R. Crameri et al. p. 128

## Description

This invention relates to DNA fragments comprising genes conferring resistance to anthracycline antibiotics, to recombinant vectors comprising such DNA fragments and to hosts transformed with the vectors.

The anthracyclines of the daunorubicin group, such as doxorubicin, carminomycin and aclavinomycin, are among the most widely employed agents in antitumoral therapy. They are polyketides produced by various strains of Streptomyces (S. peucetius, S. coeruleorubidus, S. galilaeus, S. griseus, S. griseoruber, S. viridochromogenes and S. bifurcus).

Doxorubicin is only produced by S. peucetius var. caesius. The type strain S. peucetius var. caesius IMRU 3920 (hereinafter abbreviated to "S. peucetius 3920") is publically available and is described in US-A-3 590 028. S. peucetius 3920 has been deposited at the Institute of Microbiology of the Rutger University, US, receiving the index number IMRU 3920. This strain and its mutants obtained by classical mutagenic treatments are resistant to high levels of doxorubicin.

The study of the mechanisms involved in the resistance to these substances is crucial for two main reasons:
a) There are many examples in which the genes involved in the biosynthesis of secondary metabolites are all clustered together with at least one resistance gene: for example oxytetracycline (Rhodes P M, Hunter I S, Friend E J and Warren M, 1984, Trans Biochem Soc 12, 586-587), erythromycin (Stanzak R, Matsushima P, Baltz R H and Rao R N, Biotechnology vol 4, March 1986, 229-232), tylosin (Fayerman J T, Biotechnology vol 4, Sept 1986, 786-789) and tetracenomycin (Motamedi H, Hutchinson C R, Proc Natl Acad Sci USA, vol 84, 4445-4449, 1987). Cloning the biosynthetic genes can be useful with a view to altering pathways to produce different molecules or to overcome bottlenecks present in the biosynthesis routes thus augmenting the productivity of the strain.
b) The resistance itself can be implied in the regulatory mechanisms so that changing the resistance levels (i.e. augmenting the gene dosage) the productivity of the strain can be improved. This is an old idea usually performed via the classical methods of mutagenesis and random screening, but renewed by the utilisation of rDNA methods (Craveri R and Davies J E, The Journal of Antibiotics, Jan 1986, 128-135).

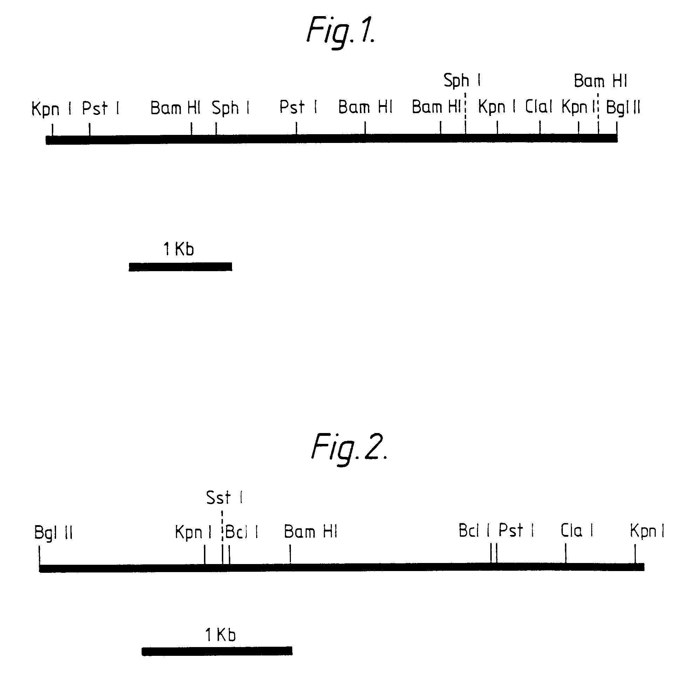

We have now isolated two DNA segments which incorporate doxorubicin resistance genes. Accordingly, the present invention provides DNA having the configuration of restriction sites shown in Figure 1 or 2 of the accompanying drawings or a restriction fragment derived therefrom containing a gene coding for doxorubicin resistance. For convenience, the DNA segments shown in Figures 1 and 2 are called here insert DNA. The invention also provides:
- recombinant vectors which are capable of transforming a host cell and which contain an insert DNA or a restriction fragment derived therefrom containing a doxorubicin resistance gene; and
- host cells transformed with such vectors.

In more detail, in the accompanying drawings:
Figure 1 is the restriction map analysis of a first DNA of the invention. This is an insert in recombinant plasmid FICE 1 (Rec 1). The insert has Sau3AI ends and was inserted into the BglII site of pIJ702. One BglII site was reconstituted after ligation.
Figure 2 is the restriction map analysis of a second DNA of the invention. This is an insert in recombinant plasmid FICE 2 (Rec 2). The insert has Sau3AI ends and was inserted into the BglII site of pIJ702. One BglII site was reconstituted after ligation.

The maps shown in Figures 1 and 2 do not necessarily provide an exhaustive listing of all restriction sites present in each DNA segment. However, the reported sites are sufficient for an unambiguous recognition of the segments.

The insert DNAs and restriction fragments of the invention contain a gene coding for doxorubicin resistance. For such a gene to be expressed, the DNA may carry its own transcription control sequence and, in particular, its own promoter which is operably connected to the gene and which is recognised by a host cell RNA polymerase. Alternatively, the insert DNA or restriction fragment may be ligated to another transcription control sequence in the correct fashion or cloned into a vector at a restriction site appropriately located neighbouring a transcription control sequence in the vector.

An insert DNA or restriction fragment carrying a doxorubicin resistance gene may be cloned into a recombinant DNA cloning vector. Any autonomously replicating and/or integrating agent comprising a DNA molecule to which one or more additional DNA segments can be added may be used. Typically, however, the vector is a plasmid. A preferred plasmid is the high copy number plasmid pIJ702 (Katz et al, J Gen Microbiol 1983 129 2703-2714). Any suitable technique may be used to insert the insert DNA or restriction fragment thereof into the vector. Insertion can be achieved by ligating the DNA into a linearised vector at an appropriate restriction site. For this, direct combination of sticky ends or homopolymer tailing or the use of a linker or adapter molecule may be employed.

The recombinant vector is used to transform a suitable host cell, typically cells that would benefit from being able to exhibit doxorubicin resistance. The host cells may be ones which are doxorubicin-sensitive, i.e. cannot grow in the presence of doxorubicin or ones which are doxorubicin-resistant but would benefit from greater resistance to doxorubicin. The host may be a microorganism. Strains of S peucetius, more particularly S peucetius var. caesius, which produce doxorubicin and other strains of Streptomyces which produce anthracyclines may therefore be transformed. Resistance, or greater resistance, to doxorubicin may enable more doxorubicin to be produced by cells of such a strain. Tolerance of greater concentrations of doxorubicin may be achieved. Transformants of strains of S peucetius are typically obtained by protoplast transformation. Doxorubicin can thus be obtained by culturing a transformed strain of S peucetius and recovering the doxorubicin thus-produced.

The insert DNAs are obtained from the genomic DNA of S peucetius M76. S peucetius M76 is a mutant of S peucetius 3920 which is able to convert daunorubicin to doxorubicin at high levels. S peucetius M76 was deposited at the Deutsche Sammlung von Mikroorganismen (DSM), Federal Republic of Germany on 11 May 1988 under accession number D.S.M. 4592. A strain derived therefrom from S peucetius M76 may also be used, which typically will also be able to convert daunorubicin to doxorubicin. Insert DNAs may therefore be obtained by:
(a) preparing a library of the genomic DNA of S peucetius M76 or a strain derived therefrom;
(b) screening the library for doxorubicin resistance;
(c) obtaining an insert DNA from a recombinant vector which forms part of the library and which has been screened as positive for doxorubicin resistance; and
(d) optionally, obtaining from the insert DNA a restriction fragment which contains a gene coding for doxorubicin resistance.

The library may be prepared in step (a) by partially digesting the genomic DNA of S peucetius M76 or a strain derived therefrom. The restriction enzyme MboI is preferably used. The fragments thus obtained can be size-fractionated. Fragments of from 4 to 6 Kb in size are preferred. These fragments are ligated into a linearised vector such as pIJ702. Host cells are transformed with the ligation mixture. Typically, the host cells are doxorubicin-sensitive, for example sensitive to 50 mcg or less or, preferably 30 mcg or less of doxorubicin per ml. For example, S lividans TK 23 protoplasts may be transformed.

In step (b), the transformants thus-obtained are screened for doxorubicin resistance. Clones doxorubicin-resistant are identified by growth in a medium containing doxorubicin. Such clones are isolated and recombinant vectors contained therein are extracted. On digestion of the recombinant vectors with suitable restriction enzymes in step (c), the S peucetius M76 DNA inserted into each vector may be identified, sized and mapped. In this way, it may be checked that the vector contains an insert DNA of the invention.

Further, two or more overlapping inserts may be isolated which are wholly or partly embraced within the DNA of the invention. These may be fused together by cleavage at a common restriction site and subsequent ligation to obtain a DNA of the invention, pared in length using appropriate restriction enzymes if necessary. Restriction fragments of an insert DNA which contains a gene encoding for doxorubicin resistance may be obtained in step (d) also by cleaving an insert DNA with an appropriate restriction enzyme.

Finally, DNA of the invention may be mutated in a way which does not affect its ability to confer doxorubicin resistance. This can be achieved via site-directed mutagenesis for example. Such mutated DNA also forms part of the invention.

The following Example illustrates the invention. In the Example Ts^{R}, Doxo^{R} and Doxo^{S} denote the thiostrepton-resistant, the doxorubicin-resistant and the doxorubicin-sensitive phenotypes respectively.

### EXAMPLE

### 1. Materials and Methods

### Bacterial strains and plasmids:

Streptomyces peucetius M76, a filamentous streptomycete producing daunorubicin and doxorubicin and resistant to doxorubicin (MIC 250 mcg/ml), and some biosynthetic mutants sensitive to doxorubicin; S. lividans TK 23 sensitive to doxorubicin.

Plasmid pIJ702 a high copy number was obtained from the John Innes Culture Collection, Norwich, GB.

### Media and Buffers

TSB contained 30 g of tryptic soy broth (DIFCO) per litre of distilled water; YEME contained 5 g of yeast extract (DIFCO), 10 g of malt extract (DIFCO), 340 g of sucrose, 5 mM MgCl₂.6H₂O and variable glycine concentrations per litre of distilled water.

The regeneration medium R2YE was as described by Chater K F, Hopwood D A , Kieser T and Thompson C J (1982) "Gene cloning in Streptomyces", 69-95 in P H Hofschneider and W Goebbel (ed) "Gene Cloning in Organisms other than E. coli", Springer-Verlag, Berlin. The medium was prepared with the following composition per litre:

| | | | |
|---|---|---|---|
| sucrose | 103 g | trace elements mix | 2 ml |
| 2.5% K₂SO₄ | 10 ml | 0.5% KH₂PO₄ | 10 ml |
| MgCl₂.6H₂O | 10.1 g | 1M CaCl₂ | 20 ml |
| glucose | 10 g | proline | 3 g |
| casaminoacids | 0.1 g | 0.25M TES pH 7.2 | 100 ml |
| agar | 22 g | 10% yeast extract | 50 ml |

Medium P was as described by Baltz R H, J Gen Microbiol 107: 93-102 (1978).

Streptomycetes were maintained on solid medium described in US-A-3 590 028, Example 2.

Growth Conditions: for liquid cultures both Streptomyces species were grown in 50 ml of YEME + TSB (1:1) at 28^{o}C on a rotary shaker at 280 rpm. The growth medium was inoculated with homogenised mycelia. Homogenisation was obtained by vortexing mycelia in a tube containing glass beads.

### Protoplast transformation

Mycelia from 35 ml of liquid culture (supplemented with 0.5% glycine) were recovered by centrifugation (10 min, 1500 x g), washed twice with 10.3% sucrose, resuspended in 10 ml of P medium containing 1 mg/ml of lysozyme (SIGMA) and incubated for 60 minutes at 30^{o}C with reciprocal shaking (280 rpm). After protoplast formation the suspension was filtered through cotton, washed once with medium P and resuspended in 1 ml of medium P. Usually 10⁸ protoplasts were obtained.

For each transformation 200 ul of medium P containing about 2 x 10⁷ protoplasts were mixed with 10 ul of the desired amount of DNA in TE (Tris-HCl 10 mM, EDTA 1 mM pH 8.0), and with 800 ul of 25% polyethylene glycol (PEG) 1000 in medium P. 1 Minute after the addition of PEG solution, transformation was terminated by the addition of 5 ml of medium P. Protoplasts were pellatted by centrifugation, resuspended in 1 ml of P and plated on R2YE. After incubation for 24 hours at 28^{o}C transformants were selected by flooding the plates with 3 ml soft NA (8 g of DIFCO nutrient broth and 5 g of agar per litre) containing the appropriate antibiotic. The number of transformants was about 1 x 10⁴ - 1 x 10⁷ per mcg of DNA, according to the strains utilised.

### Isolation of plasmid and genomic DNA

Isolation of plasmid and genomic DNA from streptomycetes was performed using techniques described by Hepwood D A et al (1985) "Genetic Manipulation of Streptomyces - A Laboratory Manual" The John Innes Foundation.

### Preparation of S. peucetius M76 genomic library

All restriction enzymes, calf thymus alkaline phosphatase and T4 ligase were obtained from BRL (Bethesda, MD) and used according to the manufacturer's instructions. S. peucetius M76 genomic DNA was partially digested with MboI, and fragments ranging between 4 and 6 Kb in size recovered by electroelution from agarose gel. These fragments were ligated to pIJ702 linearised with BglII and phosphatase treated. The ligation mixture was used to transform S lividans TK 23 protoplasts sensitive to 30 mcg/ml of doxorubicin.

### 2. Results

### Cloning of DNA fragments which confer resistance to doxorubicin in sensitive Streptomyces strains

Partially MboI digested S. peucetius M76 genomic DNA was inserted into the BglII site of pIJ702. The ligation mix was used to transform S lividans TK 23 protoplasts. Transformants were selected for Thiostrepton resistance and white colour, indicating insertional inactivation of the melanin gene of pIJ702.

Thiostrepton-resistant white colonies were then screened for resistance to doxorubicin (100 mcg/ml). They were plated on R2YE medium, incubated at 28^{o}C for 24 hours with 3 ml of soft NA containing 500 mcg/ml of doxorubicin; two clones T^{R} and Doxo^{R} were thus identified.

Extraction of plasmid DNA from these two clones revealed the presence of inserts of 5.7 kb and 4.4 kb in length. The two recombinant plasmids, named respectively FICE 1 and FICE 2, were again used to transform S lividans TK 23 protoplasts. In both cases transformation showed that the Doxo^{R} character is conferred with high efficiency along with the Ts^{R} one.

### Expression of the Doxo^{R} character in S peucetius mutants Doxo^{S}

The two recombinant plasmids were then introduced into some derivative mutants of S. peucetius M76 which are Doxo^{S} (MIC 50 ug/ml). The transformants showed complementation of the Doxo^{S} character. They could grow on doxorubicin 1500 ug/ml presenting a resistance to doxorubicin level higher than the parental strain S. peucetius M76, donor of the cloned genes (MIC 250 mcg/ml). The increased level of resistance in the transformants might be explained by the high copy number of the recombinant plasmids (pIJ101 replicon, Katz et al 1983).

### Restriction enzyme analysis of the cloned fragments

As the phenotype conferred by the two cloned fragments was the same, we investigated if there were one or two distinct functions able to confer the Doxo^{R} character. Figures 1 and 2 show the restriction maps of the S peucetius M76-derived inserts of FICE 1 and FICE 2. Most of each map is derived from the sizes of fragments generated by single and double digests using different combinations of enzymes. The interval lengths between adjacent sites come from direct measurements of the relevant fragments in appropriate double or single digests.

There is no obvious correspondence between the maps of the two cloned fragments, suggesting that the resistance is conferred by two distinct genes.

## Claims

1. A DNA having the configuration of restriction sites shown in Figure 1 or 2 of the accompanying drawings or a restriction fragment derived therefrom containing a gene coding for doxorubicin resistance.

2. A recombinant vector comprising a DNA sequence as defined in claim 1.

3. A vector according to claim 2, which is a plasmid.

4. A vector according to claim 3, wherein the DNA sequence is inserted in the plasmid pIJ702.

5. A host transformed with a vector as defined in claim 2.

6. A host according to claim 5 which is a microorganism which produces anthracyclines.

7. A host according to claim 5, which is a strain of S peucetius.

8. A process for obtaining a DNA sequence as defined in claim 1, which process comprises:
(a) preparing a library of the genomic DNA of S peucetius M76 (D.S.M. 4592) or a strain derived therefrom;
(b) screening the library for doxorubicin resistance;
(c) obtaining an insert DNA from a recombinant vector which forms part of the library and which has been screened positive for doxorubicin resistance; and
(d) optionally, obtaining from the insert DNA a restriction fragment which contains a gene coding for doxorubicin resistance.

9. A process for the preparation of a recombinant vector as defined in claim 2, which process Comprises cloning a DNA sequence as defined in claim 1 into a vector.

10. A process for the preparation of doxorubicin, which process comprises culturing a strain of S peucetius as claimed in claim 7 and recovering the doxorubicin thus-produced.

## Patentansprüche

1. DNA mit der in Figuren 1 oder 2 der anliegenden Zeichnungen gezeigten Schnittstellten-Konfiguration oder ein hiervon abgeleitetes Restriktionsfragment, enthaltend ein Doxorubicin-Resistenz-kodierendes Gen.

2. Rekombinanter Vektor, enthaltend eine DNA-Sequenz gemäß Anspruch 1.

3. Vektor gemäß Anspruch 2, welcher ein Plasmid ist.

4. Vektor gemäß Anspruch 2, worin die DNA-Sequenz in das Plasmid pIJ7002 eingefügt ist.

5. Wirt, transformiert mit einem Vektor gemäß Anspruch 2.

6. Wirt gemäß Anspruch 5, welcher ein Anthracycline-produzierender Mikroorganismus ist.

7. Wirt gemäß Anspruch 5, welcher ein Stamm von S. peucetius ist.

8. Verfahren zur Gewinnung einer DNA-Sequenz gemäß Anspruch 1, umfassend:
(a) Herstellung einer genomischen DNA-Bank von S. peucetius M76 (D.S.M. 4592) oder eines hiervon abgeleiteten Stammes;
(b) Screenen der Bank nach Doxorubicin-Restistenz;
(c) Gewinnung einer Insert-DNA aus einem rekombinanten Vektor, welcher einen Teil der Bank bildet, und welcher als positiv für Doxorubicin-Resistenz gescreent wurde; und
(d) gegebenenfalls Gewinnung eines Restriktionsfragmentes, welches ein Doxorubin-Resistenz kodierendes Gen enthält, aus der Insert-DNA.

9. Verfahren zur Herstellung eines rekombinanten Vektors gemäß Anspruch 2, umfassend die Klonierung einer DNA-Sequenz gemäß Anspruch 1 in einen Vektor.

10. Verfahren zur Herstellung von Doxorubicin, umfassend die Kultur eines S. peucetius-Stammes gemäß Anspruch 7 und Gewinnung des so hergestellten Doxorubicins.

## Revendications

1. Un ADN ayant la configuration des sites de restriction représentée par la figure 1 ou la figure 2 des dessins annexés, ou un fragment de restriction dérivé de celui-ci, contenant un gène codant pour la résistance à la doxorubicine.

2. Vecteur recombinant comprenant une séquence d'ADN telle que définie à la revendication 1.

3. Vecteur selon la revendication 2, qui est un plasmide.

4. Vecteur selon la revendication 3, dans lequel la séquence d'ADN est insérée dans le plasmide pIJ702.

5. Hôte transformé par un vecteur tel que défini à la revendication 2.

6. Hôte selon la revendication 5, qui est un micro-organisme produisant des anthracyclines.

7. Hôte selon la revendication 5, qui est une souche de S. peucetius.

8. Procédé d'obtention d'une séquence d'ADN telle que définie à la revendication 1, ledit procédé comprenant les étapes consistant :
(a) préparer une banque de l'ADN génomique de S. peucetius M76 (D.S.M. 4592) ou d'une souche dérivée de celle-ci;
(b) analyser la banque en ce qui concerne la résistance à la doxorubicine;
(c) obtenir un ADN d'insertion provenant d'un vecteur recombinant faisant partie de la banque et qui s'est avéré positif à l'analyse en ce qui concerne la résistance à la doxorubicine; et
(d) facultativement, obtenir, en partant de l'ADN d'insertion, un fragment de restriction contenant un gène codant pour la résistance à la doxorubicine.

9. Procédé pour la préparation d'un vecteur recombinant tel que défini à la revendication 2, ledit procédé comprenant le clonage d'une séquence d'ADN telle que définie à la revendication 1 dans un vecteur.

10. Procédé de préparation de la doxorubicine, ledit procédé comprenant la culture d'une souche de S. peucetius telle que revendiquée à la revendication 7 et la récupération de la doxorubicine ainsi produite.
